# EUROPÄISCHE PATENTSCHRIFT

(11) **EP 1 226 125 B1**
(45) Veröffentlichungstag und Bekanntmachung des Hinweises auf die Patenterteilung: **15.12.2004**
(21) Anmeldenummer: 00975934.1
(22) Anmeldetag: 27.10.2000
(51) Int. Cl.: C07D 235/00

(54) **VERFAHREN ZUR HERSTELLUNG VON ULKUSTHERAPEUTIKA**
METHOD FOR PRODUCING THERAPEUTIC AGENTS FOR ULCERS
PROCEDE DE FABRICATION D'AGENTS THERAPEUTIQUES CONTRE LES ULCERES

(30) Priorität: 28.10.1999 DE 19951960
(43) Veröffentlichungstag der Anmeldung: 31.07.2002
(73) Patentinhaber: Grünenthal GmbH, 52078 Aachen (DE)
(72) Erfinder: LÖBERMANN, Hartmut, 52078 Aachen (DE); CASTER, Karl-Heinz, 52249 Eschweiler (DE)
(86) Internationale Anmeldenummer: PCT/EP2000/010580
(87) Internationale Veröffentlichungsnummer: WO 2001/030765

(56) Entgegenhaltungen:
- EP-A- 0 005 129
- EP-A- 0 484 265
- EP-A- 1 000 943
- WO-A-00/02876
- WALTER W. ET AL.: "Bildung, Reaktionen, Kristall- und Molekülstruktur des cyclischen Sulfenylcarboxylates: 3-Oxo-3H-2,1-benzoxathiol-7-carbonsäuremet hylester" CHEMISCHE BERICHTE, VERLAG CHEMIE GMBH. WEINHEIM, DE, Bd. 111, 1978, Seiten 1685-1692, XP000985143
- KRISCHE B. & WALTER B.: "Cyclische Sulfenylcarboxylate aus 4H-3,1-Benzoxathiin-4-on-1-oxiden. Eine neue Ringverengungsreaktion" CHEMISCHE BERICHTE, VERLAG CHEMIE GMBH. WEINHEIM, DE, Bd. 116, Nr. 5, 1983, Seiten 1708-1727, XP000984537

## Beschreibung

Die Erfindung betrifft ein Verfahren zur Herstellung als Ulkustherapeutika geeigneter Benzimidazol-Derivate, insbesondere Omeprazol oder Pantoprazol.

Ulkustherapeutika werden heute im großen Stil zur Bekämpfung von Geschwüren, insbesondere Magengeschwüren (Ulcus ventriculi), eingesetzt. Die Ursachen für das Entstehen von Magengeschwüren sind äußerst vielfältig und es gibt eine erhebliche Anzahl von Personen, die hier auf medikamentelle Hilfe angewiesen sind. Die Behandlung erfolgt meist durch Substanzen, die die in der Magenwand lokalisierten Protonenpumpen, H⁺-K⁺-ATPasen, inhibieren. Bekannte Vertreter dieser Therapeutika sind 5-Methoxy-2-[(4-methoxy-3,5-dimethyl-2-pyridinyl)-methyl]sulfinyl-1Hbenzimidazol, generischer Name Omeprazol, und 5-(Difluormethoxy)-2-[3,4-dimethoxy-2-pyridyl)methylsulfinyl]-benzimidazol, generischer Name Pantoprazol. Insbesondere das Omeprazol ist ein bekannter Protonenpumpeninhibitor für den eine erhebliche Anzahl von Herstellungsverfahren entwickelt wurden. Die Synthese von Omeprazol und strukturell verwandter Verbindungen umfaßt typischerweise mehrere Stufen. Der letzte Schritt ist zumeist die Oxidation eines Sulfids, im Falle von Omeprazol 5-methoxy-2-[(4-methoxy-3,5-dimethyl-2-pyridinyl)-methylthio]-1H-benzimidazol, auch als Pyrmetazol bezeichnet, zum entsprechenden Sulfinyl, insbesondere zum Omeprazol. Dieser letzte Oxidationsschritt hat eine hohe Bedeutung für Ausbeute, Reinheit und auch Wirtschaftlichkeit des gesamten Herstellungsprozesses und es gibt daher im Stand der Technik verschiedene Vorschläge für diesen Syntheseschritt.

In der EP 0 005 129, in der der Stoffschutz für Omeprazol beansprucht wurde, wird die Oxidation mit Hilfe von Oxidierungsagentien wie beispielsweise m-Chlorperbenzoesäure in einem Lösungsmittel beschrieben. Dieses Lösungsmittel wird nicht weiter spezifiziert, aber die Beispiele enthalten lediglich Hinweise auf Trichlormethan, Ethanol, Benzol und Salzsäure. Die Ausbeuten und die Reinheit des Produktes waren aber nicht zufriedenstellend.

Die EP 0 533 264 offenbart eine Verfahren für die Oxidation, bei dem Magnesiummonoperoxyphthalat verwendet wird. Diese Reaktion wird üblicherweise in Lösungsmitteln durchgeführt, die Wasser, wassermischbare Lösungsmittel oder wasserunmischbare Lösungsmittel bzw. (bevorzugt) Kombinationen dieser drei Lösungsmittelarten enthalten. Es werden verschiedene Lösungsmittel aufgeführt, unter anderem niedermolekulare Alkohole als wassermischbare Lösungsmittel und Toluol als wasserunmischbares Lösungsmittel. Es werden aber weder Ketone noch explizit Aceton erwähnt und auch nicht bevorzugt.

Die EP 0 484 265 beschreibt verschiedene Möglichkeiten der Herstellung von Omeprazol, wobei der letzte Reaktionsschritt, die Oxidation von Pyrmetazol zu Omeprazol mit einer Persäure, bevorzugt m-Chlorperbenzoesäure, im sauren Medium mit Salzen des Pyrmetazols durchgeführt wird, wenn das Lösungsmittel nicht Methanol ist. Bei der bevorzugten Verwendung von Methanol hingegen wird Pyrmetazol eingesetzt und die Oxidation mit Wasserstoffperoxyd in Gegenwart eines Katalysators wie Ammoniummolybdat und einer anorganischen Base durchgeführt.

Die EP 302 720 beschreibt die Oxidation mit Wasserstoffperoxyd in Gegenwart von Vanadiumverbindungen. Hier werden als Solventien eine Reihe von Verbindungen aufgezeigt, unter denen Ethanol, Methanol, Aceton und Acetonitril bevorzugt sind. Auch wenn hier Aceton verwendet wird, ist doch erfindungswesentlich die Verwendung von Wasserstoffperoxyd mit einem Katalysator offenbart. Dies bildet den Kern des Erfindungsgedankens dieser Anmeldung.

Weiter beschreibt die GB 2 239 453 die Oxidation von Pyrmetazol durch photochemische Oxidation, indem entsprechende Verbindungen mit Licht angeregt werden, um Pyrmetazol zu Omeprazol zu oxidieren.

Die WO 98/09962 beschreibt eine Oxidation mit Peroxyessigsäure in einem zweiphasigen Medium aus Wasser und einem chlorierten organischen Lösungsmittel bei alkalischem pH. Als besonders bevorzugt ist hier Dichlormethan erwähnt.

Die WO 91/18895 entspricht dem europäischen Patent EP 0 533 752. Dieses beschreibt die Oxidation mit m-Chlorperoxybenzoesäure in einem inerten Lösungsmittel, wobei Methylenchlorid bevorzugt ist, bei einem pH um 8,0 bis 8,6, wobei der eigentliche Kern der Reaktion der Zusatz von Alkylformiat zur wässrigen Phase ist. Auch hier wird Aceton gar nicht erwähnt und prinzipiell der bereits aus dem Basispatent bekannte Weg über Chlorperoxybenzoesäure in Dichlormethan eingeschlagen.

Die WO 97/22603 offenbart ein Verfahren, in dem die letzten Reaktionsschritte alle im gleichen Lösungsmittelsystem durchgeführt werden. Dabei wird die Oxidation wieder mit m-Chlorperoxybenzoesäure durchgeführt. Als Lösungsmittelsysteme werden mit Wasser unmischbare Medien bevorzugt, beispielsweise Carbontetrachlorid, Trichlorethan, Chloroform, Methylenchlorid oder Toluol. Dabei wird besonders Toluol bevorzugt.

Die EP 240 158 bezieht sich auf Benzimidazolderivate als Ulkustherapeutika. Hier wird die Oxidation mit Perverbindungen, wie m-Chlorbenzoesäure, in halogenierten Kohlenwasserstoffen - wie Chloroform oder Dichlormethan - und/oder Alkoholen - wie Methanol, Ethanol oder Butanol - durchgeführt.

Auch die US 4,619,997 offenbart entsprechende Benzimidazolderivate, bei denen die Oxidation der Derivate mit allen bekannten Oxidationsmitteln, insbesondere Peroxysäuren, aber auch beispielsweise mit Hypochlorid-Lösung, durchgeführt wird. Die Reaktion findet vorzugsweise in inerten Lösungsmitteln, wie Benzol, Methylenchlorid oder Chloroform, statt.

Weitere hier einschlägige Schriften sind die ES 539 739, in der Iodosobenzol und Iodosotoluol als Oxidationsmittel vorgeschlagen werden, und die ES 543 816, die m-Chloroperoxybenzoesäure in Pulverform zur Oxidation vorschlägt.

Krische und Walter (Chem. Ber. 116, 1708 - 1727, 1983) beschreiben Oxidationen cyclischer Sulfide mit m-Chlorperoxybenzoesäure in Aceton.

Bereits die Vielzahl der vorgeschlagenen Verfahrensvarianten macht deutlich, daß weiterer Bedarf an Verbesserungen besteht, So weisen diese aus dem Stand der Technik bekannten Verfahren überwiegend den Nachteil auf, daß sie vielfach zu geringe Ausbeuten an insbesondere Omeprazol ergeben oder das erhaltene Omeprazol mit Ausgangs- oder Nebenprodukten verunreinigt ist. Es ist ihnen aber gemeinsam, daß, selbst wenn diese Nachteile nicht so ausgeprägt sind, alle bevorzugten oder explizit beschriebenen Herstellungsverfahren mit chlorierten organischen Lösungsmitteln wie Dichlormethan oder Trichlormethan oder anderen von einem Umwelt- und medizinischen Gesichtspunkt kritischen Verbindungen wie Toluol durchgeführt werden. Alle diese Verbindungen haben bekanntermaßen einen negativen Effekt auf die Umwelt und es besteht daher - auch im Hinblick auf eine zunehmende Verschärfung der Auflagen und damit verbundener Kosten - ein deutlicher Bedarf, hier eine Verbesserung gegenüber dem Stand der Technik zu erreichen.

Aufgabe der vorliegenden Erfindung war es daher, ein Verfahren zur Herstellung als Ulkustherapeutika geeigneter Benzimidazol-Derivate, insbesondere Omeprazol und Pantoprazol, aufzufinden, daß bei hohen Ausbeuten und großer Reinheit der Endprodukte erlaubt, auf umwelt- und gesundheitsverträglichere Lösungsmittel zurückzugreifen. Gegenstand der Anmeldung ist ein Verfahren zur Herstellung von Ulkustherapeutika gemäß Formel I: 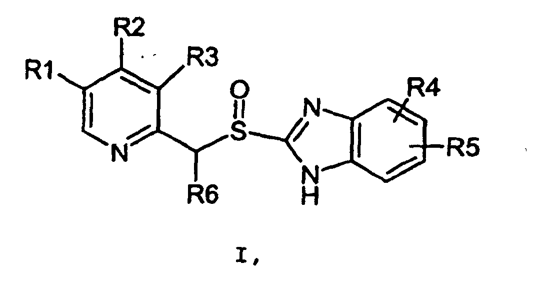 worin
R¹ , R² und R³ unabhängig voneinander ausgewählt sind aus
- Wasserstoff,
- C1-C8-Alkyl,
- C3-C8-Cykloalkyl,
- C2-C8-Fluoralkyl und
- C1-C8-Alkoxy,
R⁴ und R⁵ unabhängig voneinander ausgewählt sind aus
- Wasserstoff,
- Halogen,
- C1-C8-Alkyl,
- C3-C8-Cykloalkyl,
- CH₂C3-C8-Cykloalkyl,
- C1-C8-Alkoxycarbonyl,
- C1-C8-Alkoxy,
- C1-C8-Fluoralkoxy,
- CF₃.
- C2-C8-Fluoralkyl und
- -C(O)O-C1-C8-Alkyl und
R⁶ ausgewählt ist aus
- Wasserstoff und
- C1-C2-Alkyl,
bei dem man eine Verbindung nach Formel II: 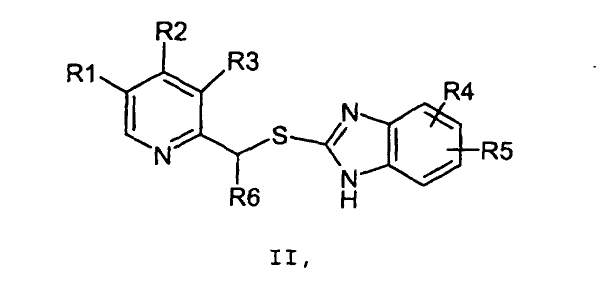 worin R¹, R², R³, R⁴, R⁵ und R⁶ die oben angegebene Bedeutung haben, mit m-Chlorperoxybenzoesäure in einem Lösungsmittel mit einem pH > 7,0 reagieren läßt und die in Anspruch 1 angegebenen Bedingungen eingehalten werden. Dabei kann gegebenenfalls ein Katalysator zugesetzt werden. Anschließend wird gegebenenfalls Wasser hinzugefügt, gegebenenfalls das Lösungsmittel entfernt und dann die Kristalle der Verbindung nach Formel I abgetrennt, wobei das genannte Lösungsmittel ein Aceton/Wassergemisch ist.

Der Vorteil dieser Verfahrensvariante gegenüber dem Stand der Technik liegt in der Verwendung von Aceton-Wasser-Gemischen als Lösungsmittel für die Oxidationsreaktion. Aceton ist ein im Vergleich zu den bisher im Stand der Technik beschriebenen und in bevorzugten Ausführungsformen - gerade für Omeprazol - genannten Lösungsmitteln als bekanntermaßen ungefährlich für die Umwelt und auch aus gesundheitlicher Sicht mit einem MAK von 1000 ppm (im Vergleich zum MAK von Toluol von 100 ppm) deutlich vorteihaft. Gleichzeitig erlauben die vorgeschlagenen Verfahren unter Verwendung von Aceton-Wasser-Gemischen als Lösungsmittel die Herstellung der Verfahrensprodukte in großer Reinheit und Ausbeute. Entsprechend vorteilhaft ist die hier vorgeschlagene Lösung.

Dabei wird als Oxidationsmittel enthaltend die Peroxygruppe m-Chlorperoxybenzoesäure eingesetzt.

Die bei den erfindungsgemäßen Verfahren gegebenfalls zugesetzten Katalysatoren können dem Fachmann bekannte Katalysatoren für Oxidationsreaktionen sein, insbesondere anorganische Salze und andere. In besonders bevorzugten Ausführungsformen der hier beanspruchten Verfahren werden aber bei der Verwendung von m-Chlorperoxybenzoesäure dem Reaktionsgemisch keine Katalysatoren zugesetzt. Unter dem Begriff Reaktionsgemisch im Sinne dieser Erfindung ist die Mischung aus einer Verbindung gemäß Formel II und dem Oxidationsmittel m-Chlorperoxybenzoesäure, in einem Aceton-Wasser-Gemisch, von pH > 7,0 zu verstehen.

Im Hinblick auf das beschriebene Verfahren wird der pH des Lösungsmittels und damit des Reaktionsgemisches durch pH-statische Titration, vorzugsweise mit NaOH, und/oder durch im Lösungsmittel gelöste oder diesem beigefügte Puffersubstanzen, vorzugsweise mono- oder dibasische Salze, insbesondere Natrium bzw. Kaliumcarbonat und/oder Natrium bzw. Kaliumbicarbonat, auf einem Wert > 7,0 gehalten. Viele der nach dem erfindungsgemäßen Verfahren herstellbaren Ulkustherapeutika, insbesondere die bevorzugten Omeprazol und Pantoprazol, sind stark säureempfindlich.

Ein ganz besonders bevorzugter Gegenstand der vorliegenden Erfindung sind erfindungsgemäße Verfahren gemäß obiger Beschreibung, bei denen in den Verbindungen gemäß Formeln I und II
R¹ = CH₃
R² = OCH₃
R³ = CH₃
R⁴= H
R⁵ = OCH₃ in 5 Position und
R⁶ = H
bedeuten. Die entsprechende Verbindung gemäß Formel I ist Omeprazol, die nach Formel II Pyrmetazol.

Ein weiterer bevorzugter Gegenstand der vorliegenden Erfindung sind erfindungsgemäße Verfahren gemäß obiger Beschreibung, bei denen in den Verbindungen gemäß Formeln I und II
R¹=H
R² = OCH₃
R³ = OCH₃
R⁴=H
R⁵ = OCF₂H in 5 Position und
R⁶=H
bedeuten.

Die entsprechende entstehende Verbindung gemäß Formel I ist Pantoprazol.

Bei der Verwendung eines Aceton-Wasser-Gemisches als Lösungsmittel im Reaktionsgemisch wird üblicherweise Wasser in einem Volumenverhältnis von 1% bis 50 % (v/v), vorzugsweise 5% bis 20 % (v/v), insbesondere 10% bis 15 % (v/v), eingesetzt.

Weiter wird das Reaktionsgemisch, insbesondere während der Oxidationsreaktion, insbesondere zur Schonung der Produkte, aber auch während des gesamten hier beschriebenen Verfahrens, zwischen -20° C und 30° C, bevorzugt zwischen -5° C und 5° C, temperiert.

Das molare Verhältnis zwischen der Verbindung nach Formel II und der m-Chlorperoxybenzoesäure beträgt bei den erfindungsgemäßen Verfahren üblicherweise 1 : 0,7 bis 1,4, vorzugsweise 1 : 0,9 bis 1,2, insbesondere 1:1.

Die fakultative Entfernung des Lösungsmittels erfolgt nach dem Fachmann gut bekannten Verfahren, wobei besonders die Entfernung des Lösungsmittels (Trocknung) unter erniedrigtem Druck, beispielsweise durch Anlegen eines Vakuums, insbesondere bei Temperaturen unterhalb der Raumtemperatur, vorzugsweise um 0°C, bevorzugt ist. Dieses Vorgehen ist besonders schonend für die Ulkustherapeutika, insbesondere für Omeprazol oder Pantoprazol.

Bevorzugterweise wird in dem erfindungsgemässen Verfahren die Entfernung des Lösungsmittels dann durchgeführt, wenn es sich um ein Aceton-Wasser-Gemisch handelt.

Im folgenden wird die Erfindung weiter durch ein Beispiel erläutert, ohne sie darauf zu beschränken.

### Beispiel

0,05 mol Pyrmetazol wurden in einem Aceton/Wasser-Gemisch, das 15 % (v/v) Wasser enthält, gelöst. Das Lösungsmittel hatte einen pH über 7,0, welcher durch die Gegenwart von 0,055 mol Natriumbicarbonat (5,5 g) aufrechterhalten wurde. Dann wurden sukzessive 0,05 mol m-Chlorperoxybenzoesäure (8,6 g) zugegeben und die Mischung zur Reaktion gebracht. Während der Zugabe und bis zum Ende der Reaktion wurde die Temperatur des Reaktionsgemisches um 0° C gehalten. Nach Zugabe von m-Chlorperoxybenzoesäure wurde zusätzliches Wasser hinzugefügt und anschließend das Lösungsmittel unter reduziertem Druck entfernt. Dabei bildete sich ein weißes kristallines Präzipitat. Die Kristalle wurden abgetrennt und mit Aceton und Wasser gewaschen. Die gewaschenen Kristalle wurden unter Vakuum getrocknet.
Ausbeute: 81 % (14,0 g)

## Patentansprüche

1. Verfahren zur Herstellung von Ulkustherapeutika gemäß Formel I: 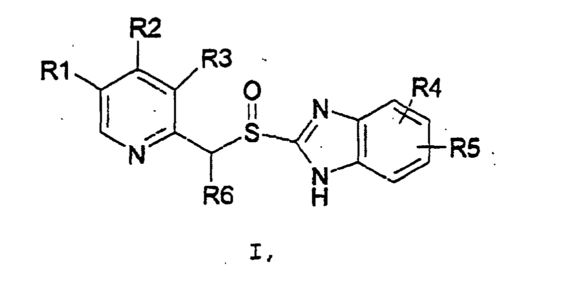 worin
R¹, R² und R³ unabhängig voneinander ausgewählt sind aus
• Wasserstoff,
• C1-C8-Alkyl,
• C3-C8-Cykloalky),
• C2-C8-Fluoroalkyl und
• C1-C8-Alkoxy,
R⁴ und R⁵ unabhängig voneinander ausgewählt sind aus
• Wasserstoff,
• C1-C8-Alkyl,
• C3-C8-Cykloalkyl,
• CH₂-C3-C8-Cykloalkyl,
• C1-C8-Alkoxycarbonyl,
• C1-C8-Alkoxy,
• C1-C8-Fluoralkoxy,
• CF₃,
• C2-C8-Fluoralkyl und
• -C(O)O-C1-C8-Alkyl und
• Halogen
R⁶ ausgewählt ist aus
• Wasserstoff und
• C1-C2-Alkyl,
bei dem man eine Verbindung nach Formel II: 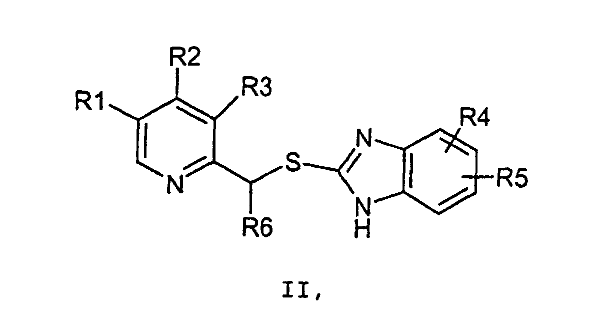 worin R¹, R², R³, R⁴, R⁵ und R⁶ die oben angegebene Bedeutung haben, mit m-Chlorperoxybenzoesäure in einem Lösungsmittel mit einem pH ≥ 7,0 reagieren läßt, anschließend gegebenenfalls Wasser hinzufügt,
gegebenenfalls das Lösungsmittel entfernt und dann die Kristalle der Verbindung nach Formel I abtrennt, **dadurch gekennzeichnet, daß** das genannte Lösungsmittel ein Aceton/Wasser-Gemisch ist, wobei der pH des Lösungsmittels durch pH-statische Titration, vorzugsweise mit NaOH, und/oder durch im Lösungsmittel gelöste oder beigefügte Puffersubstanzen, vorzugsweise mono- oder dibasische Salze, insbesondere Natrium- bzw. Kalium-Carbonat und/oder Natrium- bzw. Kalium-Bicarbonat, auf einem Wert ≥ 7,0 gehalten wird und die Temperatur des Reaktionsgemisches, insbesondere während der Reaktion zwischen der Verbindung nach Formel 11 und m Chlorperoxybenzoesäure, zwischen -20°C und 30°C, bevorzugt zwischen -5°C und 5°C gehalten wird.

2. Verfahren nach Anspruch 1, **dadurch gekennzeichnet, daß** in den Verbindungen gemäß Formeln I und II
R¹ = CH₃
R² = OCH₃
R³ = CH₃
R⁴ = H
R⁵ = OCH₃ in 5 Position und
R⁶ = H
oder
R¹ = H
R² = OCH₃
R³ = OCH₃
R⁴ = H
R⁵ = OCF₂H in 5 Position und
R⁶ = H
bedeuten.

3. Verfahren nach einem der Ansprüche 1 bis 2, **dadurch gekennzeichnet, daß** das Aceton/Wassergemisch 1% - 50% (v/v), vorzugsweise 5% - 20% (v/v), insbesondere 10% - 15% (v/v) Wasser enthält.

4. Verfahren nach einem der Ansprüche 1 bis 3, **dadurch gekennzeichnet, daß** das molare Verhältnis zwischen der Verbindung nach Formel II und m-Chlorperoxybenzoesäure 1 : 0,7 - 1,4, vorzugsweise 1 : 0,9 - 1,2, beträgt.

5. Verfahren nach einem der Ansprüche 1 bis 4, **dadurch gekennzeichnet, daß** das Aceton/Wassergemisch unter erniedrigtem Druck entfernt wird.

## Claims

1. Process for the production of antiulceratives of the formula I: 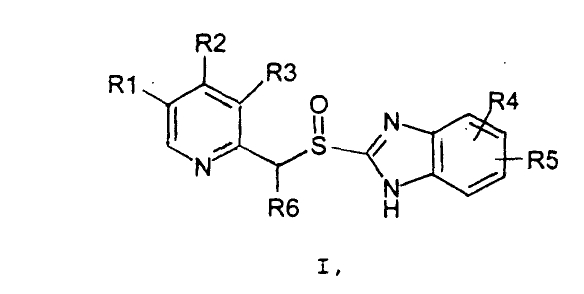 in which
R¹, R² and R³ are mutually independently selected from
• hydrogen,
• C1-C8 alkyl,
• C3-C8 cycloalkyl,
• C2-C8 fluoroalkyl and
• C1-C8 alkoxy,
R⁴ and R⁵ are mutually independently selected from
• hydrogen,
• C1-C8 alkyl,
• C3-C8 cycloalkyl,
• CH₂-C3-C8 cycloalkyl,
• C1-C8 alkoxycarbonyl,
• C1-C8 alkoxy,
• C1-C8 fluoroalkoxy,
• CF₃,
• C2-C8 fluoroalkyl and
• -C(O)O-C1-C8 alkyl, and
• halogen
R⁶ is selected from
• hydrogen and
• C1-C2 alkyl,
in which process a compound of the formula II: 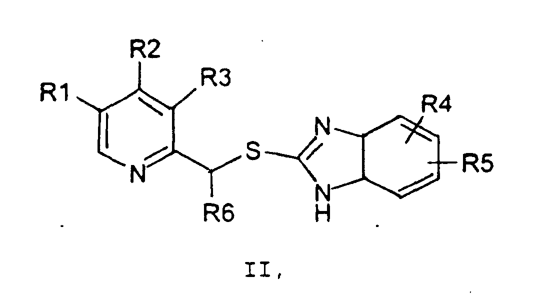 in which R¹, R², R³, R⁴, R⁵ and R⁶ have the above-stated meaning, is reacted with m-chloroperoxybenzoic acid in a solvent with a pH of ≥ 7.0, water is then optionally added, the solvent is optionally removed and then the crystals of the compound of the formula I are separated, **characterised in that** the stated solvent is an acetone/water mixture, wherein the pH of the solvent is maintained at a value of ≥ 7.0 by pH-static titration, preferably with NaOH, and/or by buffer substances dissolved in or added to the solvent, preferably mono- or dibasic salts, in particular sodium or potassium carbonate and/or sodium or potassium bicarbonate and the temperature of the reaction mixture, in particular during the reaction between the compound of the formula II and m-chloroperoxybenzoic acid, is maintained between -20°C and 30°C, preferably between -5°C and 5°C.

2. Process according to claim 1, **characterised in that** in the compounds according to the formulae I and II
R¹ means CH₃
R² means OCH₃
R³ means CH₃
R⁴ means H
R⁵ means OCH₃ in position 5 and
R⁶ means H
or
R¹ means H
R² means OCH₃
R³ means OCH₃
R⁴ means H
R⁵ means OCF₂H in position 5 and
R⁶ means H.

3. Process according to one of claims 1 to 2, **characterised in that** the acetone/water mixture contains 1%-50% (v/v), preferably 5%-20% (v/v), in particular 10%-15% (v/v) of water.

4. Process according to one of claims 1 to 3,
**characterised in that** the molar ratio between the compound of the formula II and m-chloroperoxybenzoic acid is 1:0.7-1.4, preferably 1:0.9-1.2.

5. Process according to one of claims 1 to 4, **characterised in that** the acetone/water mixture is removed under reduced pressure.

## Revendications

1. Procédé de production de médicaments contre des affections ulcéreuses de formule I : 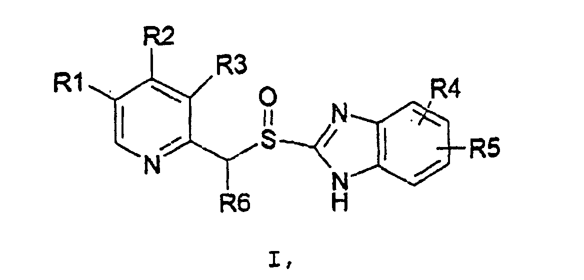 dans laquelle
R¹, R² et R³ sont choisis, indépendamment les uns des autres, entre
• hydrogène,
• alkyle en C₁ à C₈,
• cycloalkyle en C₃ à C₈,
• fluoralkyle en C₂ à C₈, et
• alkoxy en C₁ à C₈,
R⁴ et R⁵ sont choisis, indépendamment l'un de l'autre, entre
• hydrogène,
• halogène,
• alkyle en C₁ à C₈,
• cycloalkyle en C₃ à C₈,
• CH₂- (cycloalkyle en C₃ à C₈) ,
• (alkoxy en C₁ à C₈) carbonyle,
• alkoxy en C₁ à C₈,
• fluoralkoxy en C₁ à C₈,
• CF₃ ,
• fluoralkyle en C₂ à C₈ et
• -C(O)O-(alkyle en C₁ à C₈) et
R⁶ est choisi entre
• hydrogène et
• alkyle en C₁ ou C₂,
dans lequel on fait réagir un composé de formule II : 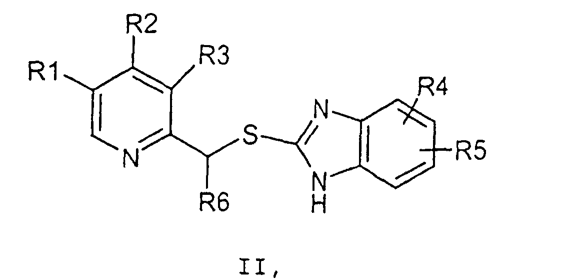 dans lequel R¹, R², R³, R⁴, R⁵ et R⁶ ont la définition indiquée ci-dessus, avec l'acide m-chloroperoxybenzoïque dans un solvant à un pH supérieur ou égal à 7,0, après quoi, le cas échéant on ajoute de l'eau, on élimine éventuellement le solvant puis on sépare les cristaux du composé de formule I, **caractérisé en ce que** le solvant mentionné est un mélange d'acétone et d'eau, le pH du solvant étant maintenu à une valeur supérieure ou égale à 7,0 par titrage statique, avantageusement avec NaOH, et/ou par des substances tampons dissoutes dans le solvant ou ajoutées au solvant, avantageusement des sels monobasiques ou dibasiques, en particulier du carbonate de sodium ou de potassium et/ou du bicarbonate de sodium ou de potassium, et la température du mélange réactionnel est maintenue entre -20°C et 30°C, avantageusement entre -5°C et 5°C, en particulier pendant la réaction entre le composé de formule II et l'acide m-chloroperoxybenzoïque.

2. Procédé suivant la revendication 1, **caractérisé en ce que** dans les composés de formules I et II,
R¹ = CH₃
R² = OCH₃
R³ = CH₃
R⁴ = H
R⁵ = OCH₃ en position 5 et
R⁶ = H
ou bien
R¹ = H
R² = OCH₃
R³ = OCH₃
R⁴ = H
R⁵ = OCF₂H en position 5 et
R⁶ = H.

3. Procédé suivant l'une des revendications 1 et 2, **caractérisé en ce que** le mélange acétone/eau contient 1 % à 50 % (v/v), avantageusement 5 % à 20 % (v/v), notamment 10 % à 15 % (v/v) d'eau.

4. Procédé suivant l'une des revendications 1 à 3, **caractérisé en ce que** le rapport molaire du composé de formule II à l'acide m-chloroperoxybenzoïque est de 1:0,7-1,4, avantageusement de 1:0,9-1,2.

5. Procédé suivant l'une des revendications 1 à 4, **caractérisé en ce que** le mélange d'acétone et d'eau est éliminé sous pression réduite.
